# EUROPEAN PATENT SPECIFICATION

(11) **EP 1 752 081 B1**
(45) Date of publication and mention of the grant of the patent: **12.10.2011**
(21) Application number: 06016460.5
(22) Date of filing: 07.08.2006
(51) Int. Cl.: A47L 15/42, A61L 2/10, A61L 2/24

(54) **A dish washer**
Geschirrspülmaschine
Lave-vaisselle

(30) Priority: 10.08.2005 KR 20050073111
(43) Date of publication of application: 14.02.2007
(73) Proprietor: LG Electronics Inc., Youngdungpo-gu Seoul (KR)
(72) Inventor: Park, Nung Seo, Seoul (KR); Yoon, Sang Heon, Gwangmyung-si Kyunggi-do (KR); Choi, Yong Jin, Goroo-ku Seoul, 152-848 (KR); Han, Dae Jong, Kangseo-ku, Seoul (KR)
(74) Representative: Vossius & Partner

(56) References cited:
- EP-A- 1 550 396
- DE-U1- 20 014 633
- GB-A- 419 929
- GB-A- 695 474
- US-A- 3 504 683
- US-A- 3 915 180
- US-A1- 2005 072 449

## Description

The present invention relates to a dish washer, particularly, to a dish washer having a sterilizing apparatus to sterilize the bacteria on the dishes.

In general, a dish washer is a home appliance that the food remnants on the dishes are washed by wash liquid which is spouted out of an injection nozzle with a high pressure.

For example, US-2005/0072449 describes a personal dishwasher which washes and dries a dish that is manually or mechanically moved through a dishwasher chamber or is manually placed in and manually removed from the chamber. Pressurized water, possibly containing soap, wetting agent, etc., may be added to the wash water. Steam may be used to for cleaning and/or disinfecting. Ultraviolet light may be used for disinfecting. The dishwasher chamber may be enlarged to pass larger dishes, bowls, glassware, etc. for washing.

US-3915180 describes a dishwasher having upper and lower utensil support racks in a washing chamber and a plurality of high energy radiating lamps on the walls of the chamber intermediate the racks for preselective heating of wash and rinse solutions in the chamber and for drying and sanitizing of utensils held in the racks by means of the lamps emitting selected infrared, visible and ultraviolet energy within the chamber.

Particularly, a dish washer includes a tub forming a washing chamber and a sump installed under the tub for storing washing liquid. The washing liquid is pushed to an injection nozzle by a washing pump installed in the sump, and then injected at a high pressure through an injection aperture formed in an end of an injection nozzle. The injected washing liquid removes dirt such as remaining food from dishes by colliding with the dishes, the removed dirt drops to a bottom of the tub.

The dish washer further includes a sterilizing apparatus for sterilizing the dishes. The sterilizing apparatus is installed on the aspect part or the rear part of the tub. The ultraviolet rays are used as a means for sterilizing the dishes.

However, if a sterilizing apparatus is installed on the aspect part or the rear part of the tub, it is onerous for users, because users have to go into the tub or disassemble the tub when a sterilizing apparatus gets out of order.

The present invention is proposed to solve the above-mentioned problems. Therefore, an object of the present invention is to provide a dish washer that the sterilizing power toward the inner part of a tub and the dishes is improved as installing the sterilizing apparatus on the inner part of a dish washer.

Another object of the present invention is to provide a dish washer which as a sterilizing apparatus is installed on a door, the users can replace and repair easily.

A dish washer according to the present invention comprises: a tub for holding dishes and opened an one end thereof; a door for selectively sealing the opened end of the tub, and have a depressed part with a little of depth on an inner side; a sterilizing apparatus which is installed on the door, wherein the sterilizing apparatus includes an ultraviolet rays generating section which occurs the ultraviolet rays, a reflection member which is installed on the depressed part, and reflects the ultraviolet rays which is occurred on an ultraviolet rays generating section into the inner side of the tub, and a cover member which prevents the influx of the washing liquid into an ultraviolet rays generating section.

A dish washer according to another aspect of the present invention comprises: a tub forming a sterilizing space that the dishes are sterilized; a door for selectively sealing the sterilizing space; a sterilizing apparatus provided on the inner side of the door; and a power supply section which supplies a power to the sterilizing apparatus, wherein the sterilizing apparatus includes an ultraviolet rays generating section which emits the ultraviolet rays into the sterilizing space, a reflection member which is formed on the inner side of the door as depressed with a little of depth, reflects the ultraviolet rays occurred on an ultraviolet rays generating section toward the sterilizing space, and a cover member united on the inner side of the door.

A dish washer according to another aspect of the present invention comprises: a sterilizing apparatus including an ultraviolet rays generating section, a supporting section which supports an ultraviolet generating section, a reflection member which reflects the ultraviolet rays occurred on the ultraviolet rays generating section, and a cover member which covers an ultraviolet rays generating section; a door that a place that the reflection member is placed is formed as depressed with a little of depth and the cover member is joined; and a tub which is able to be open by a door.

According to the present invention, it is effective that the sterilizing power of the inner part of a tub and dishes is increased as a sterilizing apparatus is placed on a door of a dish washer.

Further, according to the installing of a sterilizing apparatus on a door and be capable of being stuck or being separated of a cover member, an ultra violet rays generating section is able to be fixed or replaced easily according to a user's necessity or on the breaking down.

The accompanying drawings, which are included to provide a further understanding of the invention and are incorporated in and constitute a part of this application, illustrate embodiment(s) of the invention and together with the description serve to explain the principle of the invention. In the drawings:

Fig. 1 is a perspective view roughly showing a dish washer having a sterilizing apparatus according to the present invention.

Fig. 2 is a cross-sectional view roughly showing a dish washer having a sterilizing apparatus.

Fig. 3 is a magnified view of the part A of Fig. 2.

Reference will now be made in detail to the preferred embodiments that are concrete of the present invention, examples of which are illustrated in the accompanying drawings.

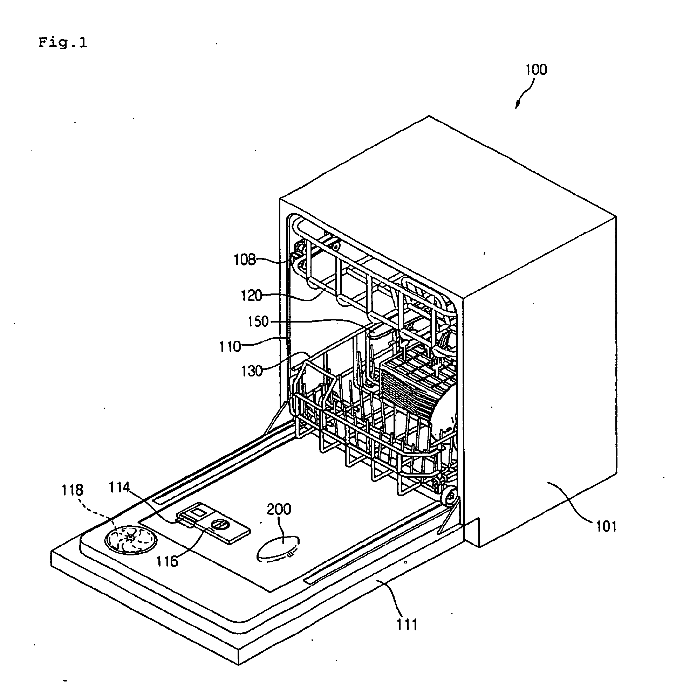

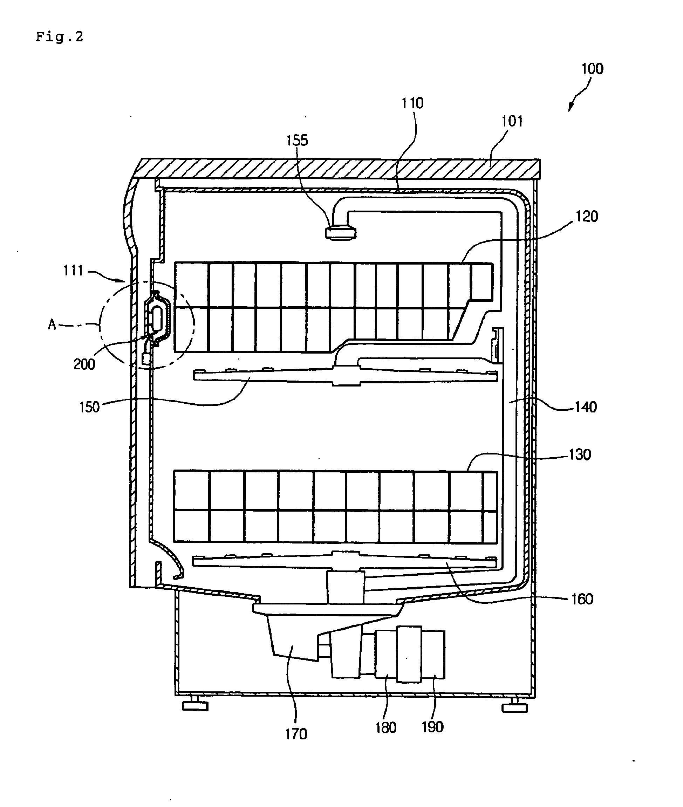

Fig. 1 is a perspective view roughly showing a dish washer having a sterilizing apparatus according to the present invention, and Fig. 2 is a cross-sectional view roughly showing a dish washer having a sterilizing apparatus.

Referring to Fig. 1 and 2, a dish washer 100 having a sterilizing apparatus according to the present invention comprises a case 101 forming the external shape, a tub 110 forming a washing chamber as placed on the inner side of the case 101, a door 111 opening and closing the washing chamber as formed on the front surface of the tub 110, and a sump 170 storing the washing liquid as formed on the low center part of the tub 110.

The dish washer 100 further comprises a washing pump 180 which is connected with the sump 170, and pumping the washing liquid which is stored on the sump 170 with a high pressure, and a washing motor 190 which is installed on the washing pump 180, and operating the washing pump 180.

The dish washer 100 further comprises a water guide 140 to be a flowing course for the washing liquid which is pumped on the washing pump 180, a lower part nozzle 160 united with the upper side of the sump 170, spouts the washing liquid out toward the upper part as formed on the low side of the washing chamber, an upper part nozzle 150 placed on the center part of the washing chamber as united perpendicular direction on the water guide 140, a top nozzle 155 provided on the end of a side of the water guide 140, and spouts the washing liquid downward perpendicularly.

The dish washer 100 further comprises a upper part rack 120 which is installed on the high side of the upper part nozzle 150 to make the dishes washed by the upper part nozzle 150, a lower part rack 130 which is installed on the high side of the lower part nozzle 160 to make the dishes washed by the lower part nozzle 160.

Particularly, the upper rack 120 is supported by the rail 108 which is placed on the inner side of the tub 110, and accomplishes the back and forth movement.

The door 111 is united with the tub 110 on the end part of the low side of the door 111 by a hinge(not shown), and is able to rotate toward the up and down directions with the hinge as centering by a user's operation. A detergent case 114 that a certain amount of the detergent is input little by little is formed on the inside of the door 111, and a rinse case 116 which make the rinse supplied during the rinsing is formed on a side of the detergent case 114.

A sterilizing apparatus 200 which sterilizes the bacteria remained on the dishes after finishing the washing stroke is formed on an inner side of the door 111.

A exhaust fan 118 is formed on a prescribed state of the door 111, so the inner part air of the tub 110 is exhausted compulsorily to the outside by the exhaust fan 118.

Hereinafter, the operations of the dish washer 100 according to the present invention will be explained.

First, a user opens the door 111 of the dish washer 100, pulls the upper part rack 120 and the lower part rack 130 toward outside of the washing chamber. And, puts the dishes on the racks 120,130. Next, closes the door 111 and approves the power, then the dish washer 100 is operated.

When the washing stroke is progressed as the power of the dish washer 100 is approved, the washing liquid is flew into the sump 170, and the washing motor 190 is operated after finishing the washing liquid flowing step. And, as an impeller(not shown) which is placed on the inner part of the washing pump 170 connected with the washing motor 190 is rotated, the washing liquid is pumped into the lower part nozzle 160 and the water guide 140.

Also, the liquid which is pumped into the water guide 140 is finally flew into the top nozzle 155 and the upper part nozzle 150, and spouted out toward the inner part of a washing chamber. And, passed through the process that the dishes accommodated on the racks 120,130 are washed by the jetted washing liquid.

In the above-mentioned, as the top nozzle 155 spouts the washing liquid downward perpendicularly and the upper part nozzle 150 spouts the washing liquid upward perpendicularly, the dishes accommodated on the upper rack 120 are washed.

As the lower part nozzle 160 spouts the washing liquid upward perpendicularly, the dishes accommodated on the lower rack 130 are washed. And, composed that the upper parts of the dishes accommodated on the lower rack 130 are washed at the same time, as a spouting outlet is formed on the lower part of the upper part nozzle 150, and the washing liquid is spouted toward the both sides of up and down.

After the washing process is finished, the foreign elements are leached out from the foul washing liquid stored on the sump 170 through a filter(not shown). The washing liquid that the foreign elements are leached out is discharged out of the dish washer 100 through a draining pump(not shown).

After the washing liquid is discharged out of the dish washer 100, the clean washing liquid is flew into the sump 170 from the outside, and spouted out through the spouting nozzles 150,160 as the same with the afore-mentioned washing process. And, the dishes are passed through the rinsing process by the spouted clean liquid.

After the rinsing process is finished, as passed through the drying process, the washing process is completed. At this time, after finishing the drying process and the washing process, the sterilizing apparatus is operated for sterilizing the bacteria which isn't sterilized as passed through the rinsing process during the custody of the dishes.

Hereinafter, the structure of the sterilizing apparatus 200 will be explained in detail.

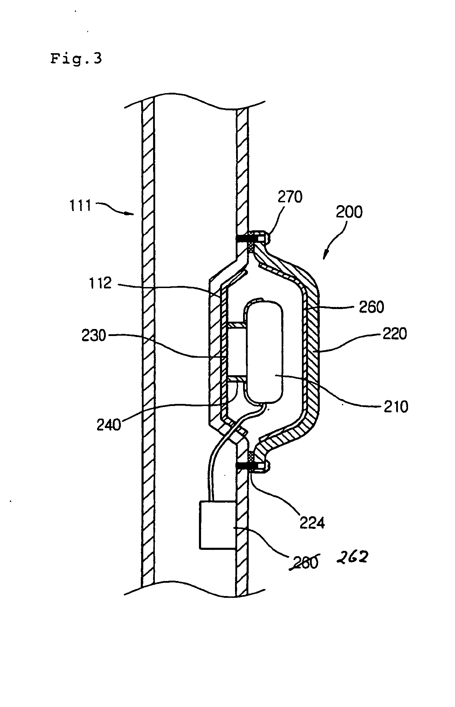

Fig. 3 is a magnified view of the part A of Fig. 2.

Referring to Fig. 3, it is distinctive that the sterilizing apparatus 200 according to the present invention is installed on the door 111 as the afore-mentioned.

Particularly, the sterilizing apparatus 200 comprises a ultraviolet rays generating section 210 which emits the ultraviolet rays into the inner part of the tub 110, so the sterilizing of the dishes, etc. is progressed, a cover member 220 which prevents the inflow of the washing liquid into the ultraviolet rays generating section 210 during the washing stroke.

In the above-mentioned, for the ultraviolet rays generating section 210, an ultraviolet rays lamp which occurs the ultraviolet rays or at least one of the LED(Light-Emitting Diode) can be used.

The ultraviolet rays generating section 210 applied the power as connected with a power supply section 260 by the electric wires placed on the inner side of the door 111.

The cover member 220 can be possessed as capable of taking out and sticking on the door 111 by an adhesion member 270 to make the ultraviolet rays generating section 210 replaced easily.

Desirably, the cover member 220 is formed as a texture that the ultraviolet rays occurred on the ultraviolet rays generating section 210 can be transmitted easily. The cover member 220 can be formed as a quartz glass texture to improve the permeability of the emitted light.

A scattering prevention section 260 can be formed as united on the inner side of the cover member 220 to prevent the scattering of the broken pieces in case that the cover member 220 is broken as being shocked. Stainless steel, iron, fiber can be used for the scattering prevention section 260, and desirably, the scattering prevention section 260 is formed as a mashed form.

A reflection member 230 is provided on the rear of the ultraviolet rays generating section 210 to improve the luminescence efficiency of the ultraviolet rays generating section 210.

That is, the reflection member 230 improves the sterilizing capacity as reflecting the ultraviolet rays which is occurred on the ultraviolet rays generating section 210 into the inner side of the tub 110. At this part, the reflection member 230 is formed as bended toward the door 111 to increase the reflecting efficiency, and according to this, a depressed part 112 as a form opposed with the reflection member 230 is formed on the inner side of the door 111.

A fixation section 240 is placed to fix the ultraviolet rays generating section 210 on the sterilizing apparatus 200. The fixation section 240 can be formed as covering the both sides of the ultraviolet rays lamp in case that the ultraviolet rays lamp is used as the ultraviolet rays generating section 210.

Therefore, the damage and injury are prevented when the door is opened or closed, as the shaking of the ultraviolet rays generating section 210 is prevented as being fixed by the fixation section 240.

At this part, the fixation section 240 can be formed separately with the reflection member 230 or the fixation section 240 and the refection member 230 can be formed a single body.

A sealer 224 which prevents the flowing of the washing liquid into the ultraviolet rays generating section 210 is placed on the contacting part of the cover 220 and the door 111.

As giving the explanations about the operations of the sterilizing apparatus according to the above-mentioned constitution: the dish washing is started as the power is supplied to the dish washer 100, and passed through the rinsing process after the washing operation is finished. At this time, the sterilizing operation for the dishes is progressed by the hot temperature washing liquid on the rinsing process. And, the dry process is passed through if the rinsing process is finished, then the washing stroke is completed.

At this time, the sterilizing apparatus 200 is operated to sterilize the bacteria which isn't sterilized during the rinsing process for the second time after the dry process or the washing stroke is completed.

Particularly, after the power is approved to the ultraviolet rays generating section 210 from the power supply section 262, the ultraviolet rays generating section 210 emits the ultraviolet rays into the inner part of the tub 110. At this time, a prescribed amount of the ultraviolet rays which is emitted on the ultraviolet rays generating section 210 improves the sterilizing efficiency as reflected into the inner part of the tub 110 by the reflection section 230.

On the other hand, in order to replace the ultraviolet rays generating section 210 according to a user's necessity or the obstacles, separate the cover member 220 from the door 111 for the first. And, as install the cover member 220 again after that on the door 111 after replacing the ultraviolet rays generating section 210, the replacement of the ultraviolet rays generating section 210 is completed.

As above-explained, it is advantageous that the ultraviolet rays generating section 210 is replaced easily as the sterilizing apparatus 200 is installed on the door 111 and the cover member 220 is formed as capable of attaching and separating on the door 111.

## Claims

1. A dish washer (100) having a tub (110) in which dishes are accommodated and a door (111) which opens and closes the tub, having a sterilizing apparatus (200) **characterized in that**
said sterilizing apparatus (200) is installed on the inside of the door (111) to sterilize inside of the tub;
wherein the sterilizing apparatus (200) includes an ultraviolet rays generating section (210) which generates the ultraviolet rays, a reflection member (230) which is formed on an inner side of the door (111) in a recessed once, reflects the ultraviolet rays generated by the ultraviolet rays generating section (210) to the tub (110), a cover member (220) which prevents the influx of the washing liquid into the ultraviolet rays generating section (210).

2. The dish washer according to claim 1, wherein the ultraviolet rays generating section (210) is an ultraviolet lamp.

3. The dish washer according to claim 1, wherein the ultraviolet rays generating section (210) is a Light-Emitting Diode.

4. The dish washer according to any one of the preceding claims, further comprising a sealing member (224) which seals the contacting part of the cover member (220) and the door (111).

5. The dish washer according to any one of the preceding claims, wherein the cover member (220) is capable of attaching and separating on the inner side of the door (111).

6. The dish washer according to any one of the preceding claims, further comprising a fixation section (240) for fixing an ultraviolet rays generating section (210).

7. The dish washer according to claim 6, wherein the fixation section (240) is formed as united on the reflection member (230).

8. The dish washer according to any one of the preceding claims, wherein the cover member (220) is formed as a transparent texture.

9. The dish washer according to any one of the preceding claims, further comprising a scattering prevention section (260) which prevents the scattering of the broken pieces when the cover member (220) is damaged, wherein the scattering prevention section (260) is formed as united on the inner side of the cover member (220).

10. The dish washer according to claim 9, wherein the scattering prevention section (260) is formed as a mashed form.

11. A dish washer according to any one of the preceding claims, further comprising a power supply section (262) which supplies a power to the sterilizing apparatus (200).

## Patentansprüche

1. Geschirrspülmaschine (100) mit einem Bottich (110), in dem das Geschirr aufgenommen wird, und einer Tür (111), die den Bottich öffnet und schließt, die eine Sterilisationsvorrichtung (200) aufweist,
**dadurch gekennzeichnet, dass**
die Sterilisationsvorrichtung (200) auf der Innenseite der Tür (111) angebracht ist, um das Innere des Bottichs zu sterilisieren;
wobei die Sterilisationsvorrichtung (200) einen Ultraviolettstrahlenerzeugungsabschnitt (210), der die Ultraviolettstrahlen erzeugt, ein Reflexionselement (230), das auf einer Innenseite der Tür (111) in einem vertieften Bereich ausgebildet ist und die durch den Ultraviolettstrahlenerzeugungsabschnitt (210) erzeugten Ultraviolettstrahlen zum Bottich (110) reflektiert, und ein Abdeckelement (220) aufweist, das den Zutritt der Waschflüssigkeit in den Ultraviolettstrahlenerzeugungsabschnitt (210) verhindert.

2. Geschirrspülmaschine nach Anspruch 1, wobei der Ultraviolettstrahlenerzeugungsabschnitt (210) eine Ultraviolettlampe ist.

3. Geschirrspülmaschine nach Anspruch 1, wobei der Ultraviolettstrahlenerzeugungsabschnitt (210) eine lichtemittierende Diode ist.

4. Geschirrspülmaschine nach einem der vorhergehenden Ansprüche, die ferner ein Dichtungselement (224) aufweist, das den Berührungsteil des Abdeckelements (220) und der Tür (111) abdichtet.

5. Geschirrspülmaschine nach einem der vorhergehenden Ansprüche, wobei das Abdeckelement (220) an der Innenseite der Tür (111) befestigt und von ihr getrennt werden kann.

6. Geschirrspülmaschine nach einem der vorhergehenden Ansprüche, die ferner einen Befestigungsabschnitt (240) zur Befestigung eines Ultraviolettstrahlenerzeugungsabschnitts (210) aufweist.

7. Geschirrspülmaschine nach Anspruch 6, wobei der Befestigungsabschnitt (240) am Reflexionselement (230) integriert ausgebildet ist.

8. Geschirrspülmaschine nach einem der vorhergehenden Ansprüche, wobei das Abdeckelement (220) als eine transparente Textur ausgebildet ist.

9. Geschirrspülmaschine nach einem der vorhergehenden Ansprüche, die ferner einen Zerstreuungsverhinderungsabschnitt (260) aufweist, der die Zerstreuung der Bruchstücke verhindert, wenn das Abdeckelement (220) beschädigt wird, wobei der Zerstreuungsverhinderungsabschnitt (260) auf der Innenseite des Abdeckelements (220) integriert ausgebildet ist.

10. Geschirrspülmaschine nach Anspruch 9, wobei der Zerstreuungsverhinderungsabschnitt (260) als Gitterform ausgebildet ist.

11. Geschirrspülmaschine nach einem der vorhergehenden Ansprüche, die ferner einen Stromversorgungsabschnitt (262) aufweist, der der Sterilisationsvorrichtung (200) Strom zuführt.

## Revendications

1. Lave-vaisselle (100) ayant une cuve (110) dans laquelle la vaisselle est reçue et une porte (111) qui ouvre et ferme la cuve, ayant un appareil de stérilisation (200), **caractérisé en ce que** ledit appareil de stérilisation (200) est installé sur la partie intérieure de la porte (111) pour stériliser l'intérieur de la cuve ;
dans lequel l'appareil de stérilisation (200) comprend une section de génération de rayons ultraviolets (210) qui génère les rayons ultraviolets, un élément réfléchissant (230) qui est formé sur un côté intérieur de la porte (111) dans une zone évidée et réfléchit les rayons ultraviolets générés par la section de génération de rayons ultraviolets (210) sur la cuve (110), un élément formant couvercle (220) qui empêche l'afflux de liquide de lavage dans la section de génération de rayons ultraviolets (210).

2. Lave-vaisselle selon la revendication 1, dans lequel la section de génération de rayons ultraviolets (210) est une lampe à rayons ultraviolets.

3. Lave-vaisselle selon la revendication 1, dans lequel la section de génération de rayons ultraviolets (210) est une diode électroluminescente.

4. Lave-vaisselle selon l'une quelconque des revendications précédentes, comprenant en outre un élément d'étanchéité (224) qui rend étanche la partie de contact de l'élément formant couvercle (220) et la porte (111).

5. Lave-vaisselle selon l'une quelconque des revendications précédentes, dans lequel l'élément formant couvercle (220) peut être fixé sur le côté intérieur de la porte (111) ou retiré du côté intérieur de la porte (111).

6. Lave-vaisselle selon l'une quelconque des revendications précédentes, comprenant en outre une section de fixation (240) pour fixer une section de génération de rayons ultraviolets (210).

7. Lave-vaisselle selon la revendication 6, dans lequel la section de fixation (240) est formée unitairement sur l'élément réfléchissant (230).

8. Lave-vaisselle selon l'une quelconque des revendications précédentes, dans lequel l'élément formant couvercle (220) est formé avec une texture transparente.

9. Lave-vaisselle selon l'une quelconque des revendications précédentes, comprenant en outre une section anti-dispersion (260) qui empêche la dispersion des morceaux cassés lorsque l'élément formant couvercle (220) est endommagé, dans lequel la section anti-dispersion (260) est formée comme une unité sur le côté intérieur de l'élément formant couvercle (220).

10. Lave-vaisselle selon la revendication 9, dans lequel la section anti-dispersion (260) a une forme écrasée.

11. Lave-vaisselle selon l'une quelconque des revendications précédentes, comprenant en outre une section d'alimentation électrique (262) qui fournit un courant à l'appareil de stérilisation (200).
